# EUROPEAN PATENT APPLICATION

(11) **EP 4 083 181 A1**
(43) Date of publication of application: **02.11.2022**
(21) Application number: 21170533.0
(22) Date of filing: 26.04.2021
(51) Int. Cl.: C12M 1/12, A61L 2/28, C12M 1/34, C12M 1/36, C12Q 1/22

(54) **STERILITY TESTING MODULE**

(71) Applicant: Sartorius Stedim Biotech GmbH, 37079 Göttingen (DE)
(72) Inventor: ARAKEL, Eric, Clement, Göttingen (DE); GLOTH, Kai, Göttingen (DE); VAN ROSSUM, Denise, Montreal (CA); GUENEC, Olivier, Hamari (FI); PRÜHL,Sebastian, Wende (DE); AUSTERJOST, Jonas, Rietberg (DE); SÖLDNER, Robert, Göttingen (DE)
(74) Representative: Gottschald Patentanwälte Partnerschaft mbB

(57) **Abstract**

The invention relates to a sterility testing module for sterility testing based on optically analysing at least one test liquid (3), which is contained in a liquid container (4). It is proposed that the sterility testing module (1) comprises a module carrier (6) and, carried by the module carrier (6), a local control unit (7) and a sensor arrangement (9) with a sensor, preferably a camera unit (10), that in an analyzing routine (8) for deriving the contamination state of the test liquid (3), the sensor arrangement (9) generates image-related data (11) representing at least one optical image (I) of the test liquid (3) and provides those image-related data (11) to the local control unit (7) and that the module carrier (6) provides a carrier interface (12), via which the module carrier (6) may be mounted to the liquid container (4), defining the position of the sensor arrangement (9) with respect to the liquid container (4).

## Description

The present invention relates to a sterility testing module according to the general part of claim1, a sterility testing assembly according to claim 15, a sterility testing arrangement according to claim 16 and a method for sterility testing according to claim 17.

Sterility testing, for which this invention was designed, is of crucial importance for a wide range of biological and/or medical liquids. For example in microbiological quality control testing it has to be ensured that sterile preparations such as vaccines are free of viable contaminating microorganisms.

Today, sterility testing is performed in one of two ways: Direct inoculation, where a growth medium (called 'test liquid') is inoculated with a test sample and incubated for a period prescribed by the pharmacopeia; or membrane-filtration. Membrane filtration involves filtering the test solution through a membrane filter enclosed within a closed system/ canister, filling it with a test liquid and incubating it for a period prescribed by the pharmacopeia. After the incubation period, the test liquid is visually inspected by an experienced laboratory professional. Any visual change, largely corresponding to turbidity, within the liquid, indicates the presence of contaminating microorganisms in the test sample.

The known setup for sterility testing (US 7,354,758 B2), which is the starting point for the invention, is based on optically analyzing at least one test liquid, which is contained in a liquid container. This known sterility testing is performed by membrane filtration. This means that after the sample liquid, that is actually to be analyzed in view of sterility, has been passed through a membrane filter, a nutrient media in the form of a nutrient solution is introduced into the liquid container, which resulting liquid is called "test liquid" in the following. After an incubation period, the test liquid is visually inspected by an experienced laboratory professional. As the distribution of known contaminants in the test liquid in most cases goes along with a corresponding turbidity within the liquid, this optical inspection is a powerful measure to find out about the contamination state of the test liquid and as a result of the sample liquid. Based on the distribution characteristics of the contaminants in the test liquid it may also be possible to find out about the specific organism causing the contamination.

Despite the routine optimization of accessories designed for sterility testing, such as, containers, filters, pumps and connectors, in view of usability and compactness, the core method still largely relies on the experience of the respective laboratory professional for the visual inspection of the test liquid. This systematically leads to a certain decrease in reproducibility, such that the known method for sterility testing is mostly conducted under the four-eyes-principle, which is cost- and time-consuming.

The problem underlying the invention is therefore to improve the known method for sterility testing such that the reproducibility is increased, without reducing the usability and compactness.

The above noted problem is solved for a sterility testing module with the features of the general part of claim 1 by the features of the characterizing part of claim 1.

First of all, it has been found that the interrelation between the distribution characteristics of the contaminants in the test liquid and the respective contamination state, which is going along with these distribution characteristics, may well be the basis for the automation of the examination of sterility testing. This is true because the distribution characteristics goes along with characteristic turbidity within the fluid, which may easily be detected by an optical sensor, preferably a camera unit, as optical contrasts, aggregation and/or sedimentation of particles, change in color and/or brightness or the like.

Second, it has been found that it is well possible to physically assign and even integrate at least part of the above noted automation into the liquid container with the test liquid to be analyzed.

In detail, a sterility testing module for sterility testing based on optically analysing at least one test liquid, which is contained in a liquid container, is proposed. According to the invention, the sterility testing module comprises a module carrier and, carried by the module carrier, a local control unit and a sensor arrangement with an optical sensor, preferably a camera unit. In an analyzing routine for deriving the contamination state of the test liquid, the sensor arrangement generates image-related data representing at least one optical image of the test liquid and provides those image-related data to the local control unit. The module carrier provides a carrier interface, via which the module carrier may be mounted to the liquid container, defining the position of the sensor arrangement with respect to the liquid container.

With the proposed solution, a sterility testing may now be performed in a fully automated and thereby fully reproducible way. In addition, at least part of the entities necessary for such automation, namely the local control unit and the sensor arrangement, may be carried by a single module carrier, which, for sterility testing, is mounted onto the liquid container. As a result, a high degree of usability may be achieved, as a complex installation of various measurement apparatus is not necessary for automated definition of the contamination state of the test liquid. Also, because the sterility testing module may be mounted directly onto the liquid container, compactness could not be any higher.

The term "image-related data" is to be understood in a broad sense. It represents at least one image of the test liquid and can in this sense be a normal photographic representation. In addition, the term "image-related data" may comprise sensor data of other sensors of the sensor arrangement regarding other properties of the test liquid, that contribute information regarding the contamination state to be derived based on the image-related data. Such properties may regard, for example, carbon-source concentration, nitrogen-source concentration, amino acid concentration, growth factors concentration, pH, temperature, oxygen concentration, carbon dioxide concentration, conductivity, pressure, DNA concentration, protein concentration, biomass concentration, biomass production rate, oxygen uptake rate and/or metabolites, such as lactic acid.

In line with this understanding, the analyzing routine, which is relying on the image-related data, is also called "image-related analyzing routine". The image-related analyzing routine may in addition include taking into account sensor data of other sensors for the derivation of the contamination state.

According to claim 2, the container interface interacts with the carrier interface in a form fit and/or force fit manner. Depending on the design of those interfaces, defining the position of the arrangement with respect to the liquid container may be realized in various ways. For example, according to claim 3, the above noted interfaces provide a locking mechanism, again in a form fit or in a force fit manner. With this, the locking mechanism may be a snap on mechanism, a bayonet mechanism, a screw mechanism or the like.

Claim 4 is directed to a preferred overall structure of the liquid container, that is particularly suitable for the proposed interaction with the module carrier. This is especially true for the preferred embodiment according to claim 5, which is directed to the liquid container being of upright design along a longitudinal axis.

Claims 6 to 9 are directed to preferred embodiments of the module carrier respective the liquid container. Especially preferred are the embodiments according to claims 7 and 8, which are directed to the module carrier being designed in the form of a cap, which further preferably provides a cover of at least part of the liquid container.

A preferred alignment of the optical sensor, in particular the camera unit, with the longitudinal axis of the liquid container is subject of claim 10. With this alignment of the optical sensor, in particular the camera unit, the viewing direction of the optical sensor, in particular the camera unit, is from top to bottom or vice versa, which results in an easy mounting of the module carrier, in particular with the above noted, preferred upright design of the liquid container.

With the further preferred embodiment according to claim 11, the image-related data generated by the sensor arrangement may regard images in different focal planes, which focal planes may be controlled by the local control unit and possibly by the external control unit. With this it is possible to analyze the test liquid not only two-dimensionally, but also three-dimensionally.

In order to further improve the reproducibility of the analyzing routine, according to claim 12, it is proposed to provide the sensor arrangement with a light arrangement for illuminating the test liquid. Preferably, this light arrangement is being controlled by the local control unit, in order to modify the lighting properties.

In one alternative of claim 13, in the analyzing routine, the local control unit derives the contamination state from the image-related data based on the interrelation between the distribution characteristics of contaminants in the test liquid and the respective contamination state. According to a second alternative in claim 13, the local control unit is in data connection with an external control unit, such that the external control unit derives the contamination state from the image-related data as noted above. Depending on the chosen alternative, the local control unit or the external control unit have to be equipped with corresponding computing performance for deriving the contamination state.

According to claim 14, the local control unit or the external control unit is designed to derive the contamination state based on a machine learning algorithm, which is trained to derive the contamination state from the image-related data, in particular from the distribution characteristics of the contaminants in the image-related data. For this, preferably, the local control unit or the external control unit are provided with a training data set, that has been generated in a training step explained below with regard to the proposed method.

Another teaching according to claim 15 is directed to a sterility testing assembly, comprising both the proposed sterility testing module and an above noted liquid container, while yet another teaching according to claim 16 is directed to a sterility testing arrangement, comprising both the proposed sterility testing module and an above noted, external control unit. All previous explanations given for the proposed module and all following explanations given for the proposed method are fully applicable to those additional teachings.

According to claim 17, a method is claimed, using the proposed sterility testing module or the proposed sterility testing assembly or the proposed sterility testing arrangement, for sterility testing based on optically analyzing at least one test liquid, which test liquid is contained in the liquid container as noted above.

According to the above noted method, in an analyzing routine, image-related data representing at least one optical image of the test liquid are being generated by the sensor arrangement, wherein the contamination state of the test liquid is derived by the local control unit or the external control unit from the image-related data based on the interrelation between the distribution characteristics of contaminants in the test liquid and the respective contamination state.

The proposed method may be realized for a wide variety of sterility testing concepts. In this context, claim 18 points out sterility testing based on membrane filtration as a first preferred alternative and sterility testing based on direct inoculation as a second preferred alternative. Other alternatives are well applicable here.

According to claims 19 to 22, prior property data are being generated in a prior analyzing routine, which is preceding the above noted, image-related analyzing routine. The term "preceding" means here, that the prior analyzing routine is performed, before the contamination state of the test liquid has been derived in the image-related analyzing routine. Accordingly, there may be a considerable overlapping taking place between those analyzing routines. The advantage is that it offers a higher level of reproducibility and reliability, since it allows for an earlier detection of potential contaminants, while simultaneously being as compliant as possible to official regulations, such as the pharmacopeia.

During the prior analyzing routine, properties of the test liquid may be obtained such as, again for example, carbon-source concentration, nitrogen-source concentration, amino acid concentration, growth factors concentration, pH, temperature, oxygen concentration, carbon dioxide concentration, conductivity, pressure, DNA concentration, protein concentration, biomass concentration, biomass production rate, oxygen uptake rate and/or metabolites, such as lactic acid.

The above may be advantageous in order to improve the reliability and/or the level of detail of the results of the image-related analyzing routine by additionally taking into account the prior property data (claim 21). In addition or as an alternative, the prior analyzing routine may provide a rapid detection of the contamination state, which is called "preliminary contamination state" in the following (claim 22). The preliminary contamination state may later be confirmed by the image-related analyzing routine. It may be pointed out, that a number of prior analyzing routines may be part of the proposed method, that each work according to one or more of claims 19 to 22.

Claim 23 is directed to different definitions of the contamination state, each going back to representing the contamination state by a contamination class out of a predefined group of contamination classes. This means that the proposed method for sterility testing then goes back to classification based on the image-related data. Compared to a quantitative definition of the contamination state, such classification is easy to realize and, in addition, completely satisfactory in view of today's standards for sterility testing.

A preferred overall alternative for the realization of the proposed method for sterility testing is subject of claims 24 to 29. Here it is proposed that the analyzing routine is based on a machine learning mechanism mentioned earlier, which is trained to derive the contamination state from the image-related data, and, if so, from the prior property data. This is a most powerful approach to achieve very good classification results even if the distribution characteristics of the contaminants varies with one and the same contaminating organism.

According to claim 26, preferably, the machine learning mechanism is based on a trained neural network, which is, further preferably, a neural convolution network (CNN). Such neural convolution networks have proven to be extremely effective for classification tasks based on images.

As an effective way to reduce the necessary computation power for the classification step, according to claim 28, it is preferred to define a region of interest within the respective image, which is subject to classification. This means, that not the whole image is being analyzed, but only the region of interest. This definition of the region of interest is done in a crop step, which may either be performed manually or automatically, for example based on feature extraction.

Claim 29 is directed to a training step for training the machine learning mechanism, which training step is preferably based on annotated images representing the contamination classes to be derived in the analyzing routine. The result is a training data set, which is used by the machine learning mechanism in the classification step. The training step may be repeated at any time, such that the training data set may be continuously improved.

All in all it is preferred, that the external control unit is performing the training step according to claim 29, generating the training data set, while the local control unit is performing the classification step according to claim 27 based on the training data set. With this approach, the local control unit may be equipped with cost effective hardware like a single chip microprocessor, while the external control unit can be a powerful computation unit, which may even make use of numerous other computation units.

In the following, an embodiment of the invention is explained with respect to the drawings. In the drawings show
- Fig. 1: a proposed sterility testing module of a sterility testing system during the analyzing routine,
- Fig. 2: the mounting of the sterility testing module of Fig. 1 onto a liquid container in subsequent steps a) and b),
- Fig. 3: a flowchart of the analyzing routine using a machine learning mechanism and
- Fig.4: a flowchart of the training step for training of the machine learning mechanism.

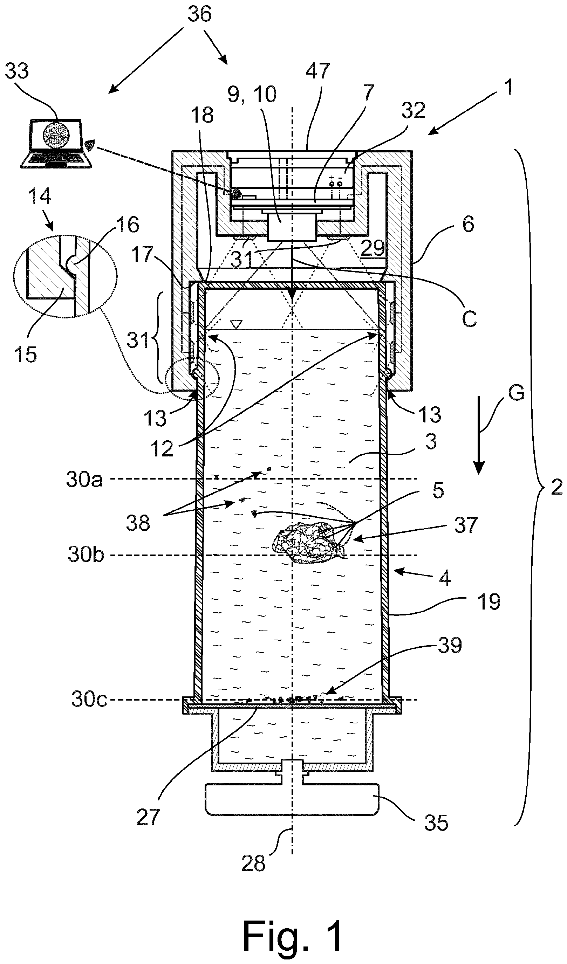

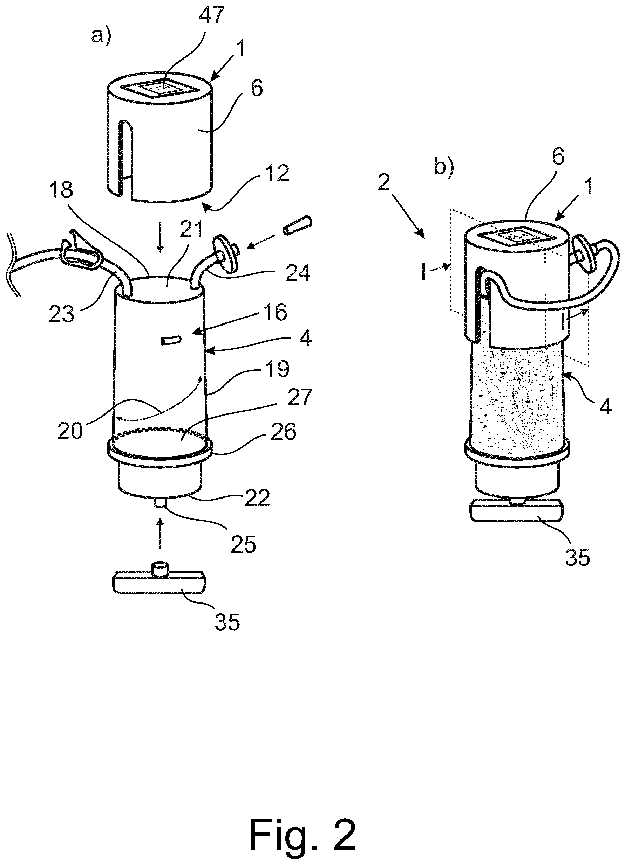

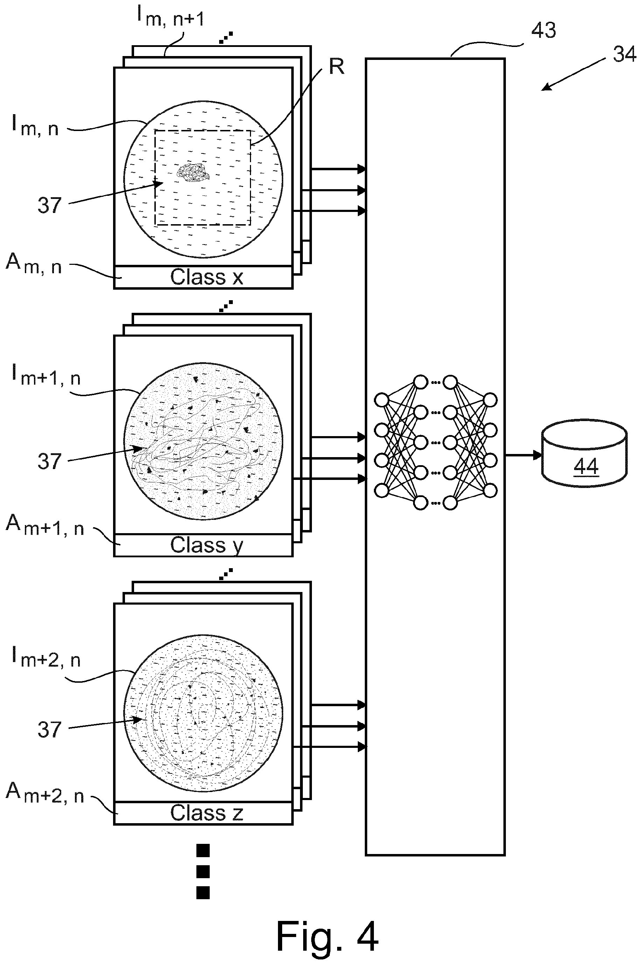

The function of the proposed sterility testing module 1 shown in Fig. 1, which is part of the sterility testing assembly 2 shown in Fig. 2b, is based on optically analyzing at least one test liquid 3, which test liquid 3 is contained in a liquid container 4. Depending on the contamination state of the test liquid 3, non-liquid contaminants 5 are distributed in the test liquid 3. Fig. 4 shows on the left side examples of the distribution of the non-liquid contaminants 5 just as rough examples.

"Optical" means any kind of optical measurement in a physical sense, including but not limited to measurements based on visible light e.g. light scattering, light absorption, such as turbidity, the respective distribution of said turbidity, and/or based on non-visible light, such as UV or infra-red, or a combination thereof. Additionally, the optical measurement can be based on a pH shift visualised by the addition of at least one pH indicator to the test liquid 3, such as phenolphthalein, bromthymol blue or methyl red.

The sterility testing module 1 comprises a module carrier 6 and, carried by the module carrier 6, a local control unit 7 and a sensor arrangement 9 with an optical sensor, here and preferably a camera unit 10, which viewing direction C is towards the test liquid 3. For the realization of the sensor, numerous variants are possible. For example, light scattering concepts, IR-sensors, UV-sensors, laser-sensors or the like may be applied. In the following, the sensor is a camera unit. All explanations given for the camera unit are fully applicable to any other variant of the sensor.

In an analyzing routine 8 for deriving the contamination state of the test liquid 3, the sensor arrangement 9 generates image-related data 11 representing at least one optical image I of the test liquid 3 and provides those image-related data to the local control unit 7. This is the basis for automated examination of sterility testing, as will be explained later.

The module carrier 6 provides a carrier interface 12, via which the module carrier 6 may be mounted to the liquid container 3, defining the position of the sensor arrangement 9 with respect to the liquid container 3. The mounting of the module carrier 6 and with it the sterile testing module 1 to the liquid container is represented by the sequence from Fig. 2a to Fig 2b.

The combination of Fig. 1 and Fig. 2 shows, that the liquid container 4 provides a container interface 13 and that during mounting, the carrier interface 12 comes into form fit and/or force fit engagement with the container interface 13. In the shown and insofar preferred embodiment, the engagement between the two interfaces 12, 13 is a combined form fit and force fit engagement.

The combination of Fig. 1 and Fig. 2 also shows, that the carrier interface 12 and the container interface 13 are designed to provide a locking mechanism 14 for locking the module carrier 6 to the liquid container 4 in the mounted state shown in Fig. 2b. Here and preferably, the locking mechanism 14 is a combined form fit and force fit mechanism, as indicated above. According to Fig. 1, the carrier interface 12 comprises a snap element 15, which is in engagement with a counter snap element 16 of the liquid container 4. During mounting the module carrier 6 to the liquid container 4, the snap element 15 snaps over the counter snap element 16, locking the module carrier 6 in the mounted position shown in Fig. 2b.

For a safe definition of the position of the sensor arrangement 9 with respect to the liquid container 4, the module carrier 6 comprises a blocking edge 17, which in the mounted state is in blocking engagement with a counter blocking edge 18 of the liquid container 4.

Alternative locking mechanisms may be realized depending on the area of application, in particular depending on the requested precision regarding the position of the sensor arrangement 9 with respect to the liquid container 4. Examples are a bayonet mechanism, a screw mechanism or the like.

As is demonstrated in Fig. 2a, the liquid container 4 comprises a container body 19 with a circumferential side 20, a top 21 and a bottom 22, which container body 19 defines a closed container volume. The liquid container 4 comprises an inlet 23 and an air vent 24 at the top 21 and an outlet 25 at the bottom 22, while also at the bottom 22, a filter receptacle 26 is located containing a filter 27, which preferably is a membrane filter.

The liquid container 4 is of upright design along a longitudinal axis 28, which allows for realizing a liquid column into the liquid container 4 with the possibility of distribution of contaminants 5 in the test liquid 3 along the longitudinal axis 28. In the mounted state, the module carrier 6 and the liquid container 4 are aligned to each other along the longitudinal axis 28 of the liquid container 4, as also shown in Fig. 1.

Here and preferably, the longitudinal axis 28 is a symmetry axis at least for the envelope of the container body 19. In preferred embodiments, in cross sectional view, the liquid container 4 and/or the module carrier 6 is/are of circular design as shown in Fig. 2 or of polygonal design.

A compact and at the same time robust design is the design of the module carrier 6 in the form of a cap with a hollow interior 29, wherein in the mounted state, preferably, the hollow interior 29 covers at least part of the liquid container 4. With such a cap like design, the carrier interface 12 may optimally be realized with low constructional effort. In this respect, preferably, the hollow interior 29 of the cap like module carrier 6 provides at least part of the carrier interface 12. Preferably, with this, the module carrier 6 may be mounted to the liquid container 4 in a sliding manner, preferably along the longitudinal axis 28 of the liquid container 4, as is demonstrated with the sequence of Fig. 2a and Fig. 2b.

In the mounted state shown in Fig. 2b, the viewing direction C of the camera unit 10 is extending through a transparent part, in particular through the top 21 of the container body 19. Accordingly, the camera unit 10 is always separated from the test liquid 3, such that no sealing is necessary between the liquid container 4 and the camera unit 10.

The camera unit 10 may be a simple 2D camera unit, which even with high resolution is a low cost component. For such a 2D camera unit, the viewing direction C is identical to the optical axis of the camera unit 10.

For the acquisition of the image-related data 11, the sensor arrangement 9 may provide an additional camera unit or a number of additional camera units with different viewing directions. In the following, to reduce complexity, only one camera unit 10 is discussed.

It may also be advantageous to provide the sensor arrangement 9 with a 3D camera unit, in order to include three dimensional image-related data into the analyzing routine 8. However, somewhat three dimensional image-related data may also be generated, if the focus of the camera unit 10 can for example be controlled by the local control unit 7. This allows for acquiring image-related data 11 for images in different focal planes. As the above noted contaminants 5 may be located at different heights with respect to the direction of gravity G and as it may be desirable to detect sedimented contaminants 5, which are mostly to be found at the bottom of the liquid container 4, at which the filter 12 is located, the acquisition of image-related data 11 for different focal planes 30a,30b,30c is particularly preferred. Three of those focal planes 30a,30b,30c are shown in Fig. 1 only as examples.

The viewing direction C of the camera unit 10, in the mounted state, preferably deviates from the longitudinal axis 28 by less than 10°. With this, the camera unit 10 can monitor the complete liquid column within the liquid container 4.

In another preferred embodiment, the sensor arrangement 9 comprises a light arrangement 31 for illuminating the test liquid 3. Here and preferably, the light arrangement 31 illuminates the test liquid 3 with light of different wave lengths and/or different intensities. Those lighting parameters are preferably controllable by the local control unit 7.

Preferably, the sterile testing module 1 carries a power supply 32 such as a chargeable battery or the like. With this, the sterile testing module 1 operates in an insofar self-sufficient way.

Here and preferably, in the analyzing routine 8, the above noted image-related data 11 are first being transferred from the sensor arrangement 9 to the local control unit 7, which may for example be a driver for the sensor arrangement 9 and which may also perform a preprocessing of the image-related data 11. To reduce complexity, those preprocessed data are presently also to be understood as image-related data 11 in the above noted sense.

In a first preferred alternative, in the analyzing routine 9, the local control unit 7 itself derives the contamination state from the image-related data 11 based on the interrelation between the distribution characteristics of contaminants 5 in the test liquid 3 and the respective contamination state. This is advantageous, as the sterile testing module 1 can then operate completely in a self-sufficient way, which increases operational flexibility.

The term "distribution characteristics" is to be understood in a broad sense and refers to the entirety of possible spatial characteristics of contaminants 5, including but not limited to the distribution of contaminating organisms, particles, liquids, gases, ions, etc.

In a second preferred alternative, in the analyzing routine 9, the local control unit 7 is in data connection with an external control unit 33, in a way, such that it is the external control unit 33, which derives the contamination state from the image-related data 11 based on the interrelation between the distribution characteristics of contaminants 5 in the test liquid 3 and the respective contamination state. This makes the realization of the local control unit 7 due to its reduced functionality particularly simple and cost effective.

The external control unit 33 may be a tablet, a personal computer or a server, which is arranged separately and remotely from, but in data connection with the local control unit 7. The data connection may be wire-based or, as indicated in Fig. 1, wireless.

In any case, the control unit, that derives contamination state from the image-related data 11 as noted above, comprises computing hardware to perform the computations necessary to perform this part of the analyzing routine 8.

Preferably, the local control unit or the external control unit is designed to derive the contamination state based on a machine learning mechanism 34, which is trained to derive the contamination state from the image-related data 11, in particular from the distribution characteristics of the contaminants 5 in the image-related data 11. This will be explained later in further detail with respect to the proposed method for sterility testing.

It may be noted at that point, that according to another teaching, the above noted sterility testing assembly 2 is claimed as such with an above noted sterility testing module 1 and with an above noted liquid container 4, wherein the module carrier 6 is mounted to the liquid container 4 via the carrier interface 12. It may also be pointed out, that a sterility testing arrangement 36 with an above noted sterility testing module 1 and with an above noted external control unit 33 is claimed as such, wherein in the analyzing routine 8, the local control unit 7 is in data connection with the external control unit 33. Regarding both additional teachings, reference may be made to all previous explanations as well to all following explanations given with regard to the proposed method for sterility testing.

The proposed method for sterility testing is based on optically analyzing at least one test liquid 3, which test liquid 3 is contained in the liquid container 4 as noted above. In an analyzing routine 8, image-related data 11 representing at least one optical image I of the test liquid 3 are being generated by the sensor arrangement 9, wherein the contamination state of the test liquid 3 is derived by the local control unit 7 or the external control unit 33 from the image-related data 11 based on the interrelation between the distribution characteristics of contaminants 5 in the test liquid and the respective contamination state.

The test liquid 3 may well be the liquid, which sterility is primarily to be tested. However, here and preferably, the test liquid 3 is only analyzed in order to find out about the sterility of a sample liquid, which is different from the test liquid 3. In order to receive the test liquid 3 in the liquid container 4, a preparation routine is preferably performed. In a first step of this preparation routine, the sample liquid, which is primarily to be tested for sterility, is introduced via the inlet 23 at the top 21 of the liquid container 4, is being passed through the filter 27 within the liquid container 4, and is being discharged via the outlet 25 at the bottom 22 of the liquid container 4. Subsequently, the outlet 25 is being closed by a plug 35. It is then preferred, that in a subsequent step of the preparation routine, the test liquid 3 in the form of a nutrient solution is introduced into the liquid container 4 via the inlet 23. This may be done after the sample liquid has been discharged from the liquid container 4 through the filter 27 and, preferably, after a washing cycle. Then, the inlet 23 is being closed, here and preferably by connecting the inlet 23 to an air vent 24 of the liquid container 4. The filter 27 is preferably a membrane filter as noted above. However, other variants for the filter 27 are possible, depending on the field of application.

The above noted concept for sterility testing is based on membrane filtration as noted above, which is only one preferred alternative. A second preferred alternative is the method for sterility testing based on direct inoculation. In this case, in the preparation routine, the test liquid 3 in the form of a combination of the sample liquid, which is primarily to be tested for sterility, and a nutrient solution is introduced into the liquid container 4 directly.

The test liquid 3 together with the filter 27 communicating with the test liquid 3 are being inserted into an incubation unit, not shown. After a predetermined incubation period, which may be 14 days for example, the proposed analyzing routine 8 is preferably being performed.

The distribution of the contaminants 5 in the test liquid 3 is in most cases represented by a certain turbidity 37, as well as by the distribution of the turbidity. In some cases, in addition, the distribution is represented by the occurrence of particle aggregates 38, 39. Additionally, the distribution of the contaminants 5 in the test liquid 3 is represented by the detection and/or distribution of any solid, liquid or gaseous contaminant 5 based on measurements by laser or light beams. The frequency of the light on which the measurement is based, can be from the visible and/or non-visible spectrum, in particular from the UV or infra-red spectrum.

In the first case, the distribution of the contaminants 5 in the test liquid 3 is preferably represented by the intensity of turbidity 37 and/or the spreading of turbidity 37 and/or the geometric structure of turbidity 37 in the test liquid 3. In addition or as an alternative, as noted above, the distribution of the contaminants 5 in the test liquid 3 is represented by the occurrence of particle aggregates 38 and/or by the occurrence of sedimented particle aggregates 39. Depending on the sensor arrangement 9, at least part of those representations of the distribution of the contaminants 5 may be detected. In case of a camera based sensor arrangement 9, there is a high potential for the detection of at least turbidity 37, as an area of turbidity 37 is distinctive from an area without turbidity 37 by contrast and/or color in the image of the test liquid 3.

In addition, in a prior analyzing routine preceding the above noted, image-related analyzing routine, prior property data representing at least one property of the test liquid 3 may be generated by the sensor arrangement 9. Preferably, the prior property data are being generated in the prior analyzing routine by at least one sensor of the sensor arrangement 9, which is/are different from the at least one sensor of the sensor arrangement 9, by which the image-related data are being generated.

The above noted, prior analyzing routine may contribute to a high reliability and/or high level of detail of the contamination state derived in the image-related analyzing routine. For this, the contamination state of the test liquid 3 is derived from the image-related data 11 and in addition from the prior property data based on the interrelation between the distribution characteristics of contaminants 5 in the test liquid 3 during the analyzing routine, the properties of the test liquid 3 during the prior analyzing routine and the respective contamination state.

In addition or as an alternative, the prior analyzing routine may serve to acquire an early indication of contamination. For this, during the prior analyzing routine, a preliminary contamination state of the test liquid 3 is derived from the prior property data based on the interrelation between properties of the test liquid 3 during the prior analyzing routine and the respective preliminary contamination state.

An example for an advantageous synopsis of the image-related data 11 during the image-related analyzing routine 9 and the prior property data during the prior analyzing routine will be given in the following for a contamination, which is based on the specific organism Staphylococcus aureus.

Staphylococcus aureus exhibits a golden-yellow color caused by staphyloxanthin, a carotenoid, which these bacteria produce as antioxidant. This color can be optically analyzed based on the generated image-related data, e.g. via photometrical analysis, light absorption and/or camera-based analysis. In the image-related analyzing routine 8, this property may be detected, wherein the derived preliminary contamination is generally "contaminated". However, it is not possible in this image-related analyzing routine 8 based on the optical image alone, to derive a contamination class representing the specific contaminating organism, since other organisms also produce carotenoids.

On the other hand, Staphylococcus aureus is able to produce lactic acid on anaerobic conditions. Lactic acid can be analyzed, for example, based on the generated prior property data, e.g. via a metabolite sensor measuring lactic acid. In the prior analyzing routine, this may lead to the derivation of a preliminary contamination state of the contamination class "contaminated". As the prior analyzing routine may be performed in an early stage preceding the image-related analyzing routine 8, the preliminary contamination state may be provided accordingly early, namely before the image-related analyzing routine 8 is completed. However, it is again not possible to derive a contamination class representing the specific contaminating organism, since other organisms also produce lactic acid.

During the image-related analyzing routine 8, by a correlation of both, image-related data and prior property data, it is possible to derive a contamination class representing the specific contaminating organism, since both features in synopsis unambiguously identify the respective specific organism.

Just as a matter of completeness it may be pointed out, that the above noted lactic acid may also be detected during the image-related analyzing routine 8, as this image-related analyzing routine 8 is not necessarily restricted to image data in the narrow optical sense. In this case, the benefit of the early derivation of a preliminary contamination state would not be realized.

Additionally, identifying compounds may be added to the test liquid 3 before the prior analyzing routine and/or the analyzing routine 8 are initiated. Such identifying compound may further increase the level of accuracy in deriving a contamination class representing the specific contaminating organism. Exemplary identifying compounds may be proteins, in particular enzymes such as coagulase, oxidase or catalase, dyes such as N, N-Dimethyl-p-phenylendiammoniumchloride, indicators and/or ions,
The term "identifying compounds" refers to any physical, chemical or biological compound supporting the identification of specific organisms. As an example, Staphylococcus aureus produces the protein coagulase, which is able to react with fibrinogen, a glycoprotein. In case fibrinogen is added to a test liquid 3 containing Staphylococcus aureus, this reaction leads to a coagulation of fibrinogen exhibiting turbidity and aggregates, which are again optically analyzable, hence further supporting the identification of the specific organism.

There are various ways possible to initiate the prior analyzing routine. The easiest way is to have the user manually initiate the prior analyzing routine. However, the initiation of the prior analyzing routine may well be performed automatically, in particular, based on a trigger event. This trigger event may be the start, the middle and/or the end of the incubation period, which may easily be monitored based on time measurement. In addition, the prior analyzing routine may be initiated continuously and/or periodically during the incubation period, in order to take dynamic changes in the distribution of the contaminants 5 into account. Here and preferably, the prior analyzing routine is executed prior to the analyzing routine.

Further, there are various possible ways to define the contamination state of the test liquid 3. Here and preferably, the contamination state is defined such that it is represented by a contamination class out of a predefined group of contamination classes, wherein in the analyzing routine 8, the contamination state is assigned one of those contamination classes.

In the easiest case, the predefined group of contamination classes only includes the contamination classes "contaminated" and "non-contaminated". As an alternative, the predefined group of contamination classes includes contamination classes each representing a specific organism such as Staphylococcus aureus, Bacillus subtilis, Pseudomonas aeruginosa or Kocuria rhizophila, Clostridium sporogenes or Bacteroides vulgatus, Candida albicans or Aspergillus niger.

These organisms, taken from the current version of the pharmacopeia, are just to be understood as an example. Further organisms, such as environmental isolates and/or such not listed in the pharmacopeia, are possible as well. Such contaminating organisms may come in homogeneous or heterogeneous groups. In case of a heterogeneous group the contamination state is represented by the contamination class, to which it essentially corresponds.

During sterility testings in the quality control of the manufacturing of biopharmaceuticals official regulations require sterility testings for specific organisms, preferably specific organism classes. The exemplary organisms mentioned above represent the major species tested for during sterility testings, although further organisms, preferably further organism classes are possible.

The distribution characteristics of the contamination class "Staphylococcus aureus" is mostly reflected by the occurance of particle aggregates and/or by the occurance of sedimented particles or particle aggregates. The distribution characteristics of the contamination class "Bacillus subtilis" is mostly reflected by the occurance of particle aggregates and/or by the occurance of a heterogeneous turbidity. The distribution characteristics of the contamination class "Pseudomonas aeruginosa or Kocuria rhizophila" is mostly reflected by the occurance of a, preferably homogeneous, turbidity. The distribution characteristics of the contamination class "Clostridium sporogenes or Bacteroides vulgatus" is mostly reflected by the occurance of a homogeneous turbidity. The distribution characteristics of the contamination class "Candida albicans" is mostly reflected by the occurance of a particle film, in particular at the bottom of the liquid container. The distribution characteristics of the contamination class "Aspergillus niger" is mostly reflected by the occurance of spatially confined particle clouds and/or by the occurance of sedimented spatially confined particle clouds.

Looking at the examples for the distribution of the non-liquid contaminants 5 given on the left side of Fig. 4, it becomes apparent, that the contamination class of the contamination state may generally be derived from the image-related data 11 by simple image processing based on analysis criteria, which are assigned to the respective contamination class. Especially for a low number of contamination classes to be detected, this is an effective approach, which may be based on simple feature extraction.

However, the more contamination classes are to be detected and the more variability of the distribution characteristics within one single contamination class is to be expected, this approach may lead to a very complex catalog of analysis criteria. In this case, the application of a machine learning mechanism 33 appears to be a more powerful approach.

If the prior property data are included in the machine learning mechanism 34, the reliability and the level of detail may be enhanced during the image-related analyzing routine 8. Accordingly, it is preferably provided, that the image-related analyzing routine 8 is based on a machine learning mechanism 34, which is trained to derive the contamination state not only from the image-related data 11, in particular from the distribution characteristics of the contaminants 5 in the image-related data 11, but also from the prior property data.

According to a preferred approach, the analyzing routine 8 is based on an above noted machine learning mechanism 34, which is trained to derive the contamination state from the image-related data 11, in particular from the distribution characteristics of the contaminants 5 in the image-related data 11. Preferably, the machine learning mechanism 34 is based on a trained neural network. Particularly preferred is the application of a neural convolution network (CNN), as this has been proven to be very effective for analyzing images as noted above as well.

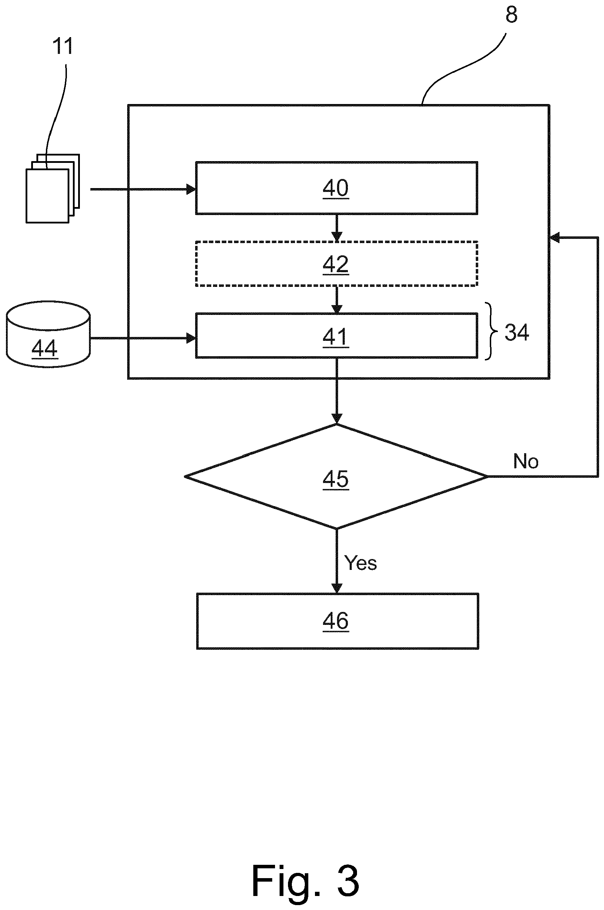

The analyzing routine 8 is shown in Fig. 3 as an example. After the transmittal of the image-related data 11 from the sensor arrangement 9 to the local control unit 7 in an acquisition step 40, a classification step 41 is performed by one of the control units 7, 33, in which the contamination class of the contamination state is derived by the machine learning mechanism 34 from the image-related data 11. This is because the machine learning mechanism 34 has been trained to derive the contamination state from the image-related data 11 as noted above and as will be explained later.

In order to reduce the computation power necessary to perform the classification step 41, a crop step 42 may be provided directly after the image-related data 11 have been received from the sensor arrangement 9 in the acquisition step 40. In the crop step 42, a region of interest R is defined in the image-related data 11, which region of interest R is subject of the classification step 41. In other words, the classification step 41 is only performed within the region of interest R, which reduces the complexity of the classification step 41. The region of interest R may well be a predefined area of the image represented by the image-related data 11. This is indicated for the image I_{1,n} in Fig. 4 as an example. With this, for example, unwanted optical reflections, which reflections might occur due to the individual geometrical structure of the sterility testing module 1, can systematically be disregarded.

As an alternative, the region of interest R may be defined by a user input. As another alternative, the region of interest R may automatically be defined by one of the control units 7, 33 based on image processing, for example based on automatic feature extraction. Other alternatives for the definition of the region of interest R are possible.

As noted above, the machine learning mechanism 34 is being trained or has been trained in a training step 43. The concept of the training step 43 is shown in Fig. 4. According to this, the training step 43 is preferably based on annotated images I_{m,n} representing the contamination classes to be derived in the analyzing routine 8. On the left side of Fig. 4, the annotated images I_{m,n} are provided, which are each assigned an annotation A_{m,n}. This annotation A_{m,n} is being assigned to the respective image manually by a laboratory professional. Based on those annotated images I_{m,n}, a training data set 44 is generated by one of the control units 7, 33 or any other control system. In the analyzing routine 8, the classification step 41 is based on the training data set 44 as is indicated in Fig. 3.

The above noted training step 43 is preferably performed by the external control unit 33 or any other control system, as for this step, considerable computation power is necessary. The resulting training data set 44 may then be downloaded into the respective control unit 7, 33, preferably into the local control unit 7, for performing the analyzing routine 8.

Depending on the contamination state, which is checked in step 45 in view of predefined reaction criteria, it may be provided, that a reaction routine 46 is initiated by one of the control units 7, 33. Such reaction criteria may be directed to whether any contamination has been detected at all in the classification step 41. Preferably, the reaction routine 46 is then the output of an optical or an acoustic alert signal. It may also be advantageous, to create an electronic alert, which is either directly communicating the detection event to the user via a display 47, that preferably is integrated into the module carrier 6 as shown in Fig. 2, or indirectly by sending the alert information to a central control system or the like.

There are various ways possible to initiate the analyzing routine 8. The easiest way is to have the user manually initiate the analyzing routine 8. However, the initiation of the analyzing routine 8 may well be performed automatically, in particular based on a trigger event. This trigger event may be end of the incubation period, which may easily be monitored by one of the control units 7, 33 based on time measurement. In addition, the analyzing routine 8 may be initiated continuously and/or periodically during the incubation period, in order to take dynamic changes in the distribution of the contaminants 5 into account.

For maximum traceability of the analysis of different test liquids 3, it is preferred, that the liquid container 4 comprises a barcode, which may be read by the camera unit 10 and transferred to one of the control units 7, 33. This double function of the camera unit 10 leads to a compact and cost effective solution.

## Claims

1. Sterility testing module for sterility testing based on optically analysing at least one test liquid (3), which is contained in a liquid container (4),
**characterized in**
**that** the sterility testing module (1) comprises a module carrier (6) and, carried by the module carrier (6), a local control unit (7) and a sensor arrangement (9) with an optical sensor, preferably a camera unit (10), that in an analyzing routine (8) for deriving the contamination state of the test liquid (3), the sensor arrangement (9) generates image-related data (11) representing at least one optical image (I) of the test liquid (3) and provides those image-related data (11) to the local control unit (7) and that the module carrier (6) provides a carrier interface (12), via which the module carrier (6) may be mounted to the liquid container (4), defining the position of the sensor arrangement (9) with respect to the liquid container (4).

2. Sterility testing module according to claim 1, **characterized in that** the liquid container (4) provides a container interface (13) and that during mounting, the carrier interface (12) comes into form fit and/or force fit engagement with the container interface (13).

3. Sterility testing module according to claim 1 or 2, **characterized in that** the carrier interface (12) and the container interface (13) are designed to provide a locking mechanism (14) for locking the module carrier (6) to the liquid container (4) in the mounted state, preferably, that the locking mechanism (14) is a form fit or a force fit mechanism.

4. Sterility testing module according to any one of the preceding claims, **characterized in that** the liquid container (4) comprises a container body (19) with a circumferential side (20), a top (21) and a bottom (22), which container body (19) defines a closed container volume, preferably, that the liquid container (4) comprises an inlet (23) at the top (21) and an outlet (25) at the bottom (22), further preferably, that a filter receptacle (26) is located at the bottom (22) of the liquid container (4).

5. Sterility testing module according to any one of the preceding claims, **characterized in that** the liquid container (4) is of upright design along a longitudinal axis (28), preferably, that in the mounted state, the module carrier (6) and the liquid container (4) are aligned to each other along the longitudinal axis (28) of the liquid container (4).

6. Sterility testing module according to any one of the preceding claims, **characterized in that** in cross sectional view, the liquid container (4) and/or the module carrier (6) is/are of circular or polygonal design.

7. Sterility testing module according to any one of the preceding claims, **characterized in that** the module carrier (6) is in the form of a cap with a hollow interior (29), preferably, that in the mounted state, the hollow interior (29) covers at least part of the liquid container (4).

8. Sterility testing module according to claim 7, **characterized in that** the hollow interior (29) of the cap provides at least part of the carrier interface (12), such that the module carrier (6) may be mounted to the liquid container (4) in a sliding manner, preferably along the longitudinal axis (28) of the liquid container (4).

9. Sterility testing module according to any one of the preceding claims, **characterized in that** in the mounted state, the viewing direction (C) of the optical sensor, in particular the camera unit (10), is extending through a transparent part, in particular through the top (21), of the container body (19).

10. Sterility testing module according to any one of the preceding claims, **characterized in that** in the mounted state, the viewing direction of the optical sensor, in particular the camera unit (10), deviates from the longitudinal axis (28) of the liquid container (4) by less than 10º.

11. Sterility testing module according to any one of the preceding claims, **characterized in that** the focus of the sensor, in particular the camera unit (10), may be controlled by the local control unit (7).

12. Sterility testing module according to any one of the preceding claims, **characterized in that** the sensor arrangement (9) comprises a light arrangement (31) for illuminating the test liquid (3), preferably, that the light arrangement (31) illuminates the test liquid (3) with light of different wave lengths and/or different intensities, preferably, that the light arrangement (31) is being controlled by the local control unit (7).

13. Sterility testing module according to any one of the preceding claims, **characterized in that** in the analyzing routine (8), the local control unit (7) derives the contamination state from the image-related data (11) based on the interrelation between the distribution characteristics of contaminants (5) in the test liquid (3) and the respective contamination state, or, that in the analyzing routine (8), the local control unit (7) is in data connection with an external control unit (33), such that the external control unit (33) derives the contamination state from the image-related data (11) based on the interrelation between the distribution characteristics of contaminants (5) in the test liquid (3) and the respective contamination state.

14. Sterility testing module according to any one of the preceding claims, **characterized in that** the local control unit (7) or the external control unit (33) is designed to derive the contamination state based on a machine learning mechanism (34), which is trained to derive the contamination state from the image-related data (11), in particular from the distribution characteristics of the contaminants (5) in the image-related data (11).

15. Sterility testing assembly with a sterility testing module (1) according to any one of the preceding claims and with a liquid container (4), wherein the module carrier (6) is mounted to the liquid container (4) via the carrier interface (12).

16. Sterility testing arrangement with a sterility testing module (1) according to any one of the claims 1 to 14 and with an external control unit (33), wherein in the analyzing routine, the local control unit (7) is in data connection with the external control unit (33).

17. Method for sterility testing for sterility testing based on optically analyzing at least one test liquid (3), which test liquid (3) is contained in a liquid container (4), using the sterility testing module (1) according to any one of the claims 1 to 14 or the sterility testing assembly (2) according to claim 15 or the sterility testing arrangement (36) according to claim 16,
wherein in an analyzing routine (8), image-related data (11) representing at least one optical image (I) of the test liquid (3) are being generated by the sensor arrangement (9) and wherein the contamination state of the test liquid (3) is derived by the local control unit (7) or the external control unit (33) from the image-related data (11) based on the interrelation between the distribution characteristics of contaminants (5) in the test liquid (3) and the respective contamination state.

18. Method according to claim 17, **characterized in that** preceding the analyzing routine (8), in order to receive the test liquid (3) in the liquid container (4), a preparation routine is performed, preferably, that in a first step of the preparation routine, a sample liquid to be tested for sterility is being passed through a filter (27), in particular a membrane filter, which filter (27) is located within the liquid container (4), and that in a subsequent step of the preparation routine, the test liquid (3) in the form of a nutrient solution is introduced into the liquid container (4), or, that in the preparation routine, the test liquid (3) in the form of a combination of the sample liquid to be tested for sterility and a nutrient solution is introduced into the liquid container (4).

19. Method according to claim 17 or 18, **characterized in that** in a prior analyzing routine preceding the analyzing routine (8), prior property data representing at least one property of the test liquid (3) are being generated by the sensor arrangement (9).

20. Method according to claim 19, **characterized in that** the prior property data are being generated in the prior analyzing routine by at least one sensor of the sensor arrangement (9), which is/are different from the at least one sensor of the sensor arrangement (9), by which the image-related data (11) are being generated.

21. Method according to claim 19 or 20, **characterized in that** the contamination state of the test liquid (3) is derived by the control unit (7) from the image-related data (11) and the prior property data based on the interrelation between the distribution characteristics of contaminants (5) in the test liquid (3) during the analyzing routine (8), the properties of the test liquid (3) during the prior analyzing routine and the respective contamination state.

22. Method according to any one of the claims 19 to 21, **characterized in that** during the prior analyzing routine, a preliminary contamination state of the test liquid (3) is derived by the control unit (6) from the prior property data based on the interrelation between properties of the test liquid (3) during the prior analyzing routine and the respective preliminary contamination state.

23. Method according to any one of the claims 17 to 22, **characterized in that** the contamination state is represented by a contamination class out of a predefined group of contamination classes and that in the analyzing routine (8), the contamination state is assigned one of said contamination classes, preferably, that the predefined group of contamination classes only includes the contamination classes "contaminated" and "non-contaminated", or, that the predefined group of contamination classes includes contamination classes each representing a specific organism such as Staphylococcus aureus, Bacillus subtilis, Pseudomonas aeruginosa or Kocuria rhizophila, Clostridium sporogenes or Bacteroides vulgatus, Candida albicans or Aspergillus niger.

24. Method according to any one of the claims 17 to 23, **characterized in that** the analyzing routine (8) is based on a machine learning mechanism (34), which is trained to derive the contamination state from the image-related data (11), in particular from the distribution characteristics of the contaminants (5) in the image-related data (11).

25. Method according to any one of the preceding claims, **characterized in that** the analyzing routine (8) is based on a machine learning mechanism (34), which is trained to derive the contamination state from the image-related data (11), in particular from the distribution characteristics of the contaminants (5) in the image-related data (11), and from the prior property data.

26. Method according to claim 24 or 25, **characterized in that** the machine learning mechanism (34) is based on a trained neural network, preferably on a neural convolution network (CNN).

27. Method according to any one of the claims 24 to 26, **characterized in that** the analyzing routine (8) comprises a classification step (41), in which the contamination class of the contamination state is derived by the machine learning mechanism (34) from the image-related data (11).

28. Method according to any one of the claims 24 to 27, **characterized in that** in a crop step (42), a region of interest (R) is defined in the image-related data (11), which region of interest (R) is subject of the classification step (41), preferably, that the region of interest (R) is a predefined area of the image (I) represented by the image-related data (11) or that the region of interest (R) is defined by a user input or that the region of interest (R) is automatically defined by one of the control units (7, 33) based on image processing, preferably based on automatic feature extraction.

29. Method according to any one of the claims 24 to 28, **characterized in that** the machine learning mechanism (34) is being trained or has been trained in a training step (43), preferably based on annotated images (I_{m,n}) representing the contamination classes to be derived in the analyzing routine (8), further preferably, that in the training step (43), a training data set (44) is generated by one of the control units (7, 33), wherein the classification step (41) in the analyzing routine (8) is based on the training data set (44).
